# EUROPEAN PATENT SPECIFICATION

(11) **EP 0 302 734 B1**
(45) Date of publication and mention of the grant of the patent: **20.10.1993**
(21) Application number: 88307234.0
(22) Date of filing: 05.08.1988
(51) Int. Cl.: A61K 37/14, A61K 39/395

(54) **Continuous processes for modifying biologically active materials and the products therefrom**
Kontinuierliche Verfahren zur Abänderung von biologisch wirksamen Substanzen und dadurch erhaltene Produkte
Procédé en continu pour la modification de substances biologiques actives et produits obtenus

(30) Priority: 06.08.1987 US 104858
(43) Date of publication of application: 08.02.1989
(73) Proprietor: OXYGENETICS INC., Santa Ana, CA 92705 (US)
(72) Inventor: Lofink, George J., Laguna Niguel 92677 (CA); Hoskins, Michael K., Orange 92669 (CA); Vail, Martha A., Ventura 93001 (CA); Schultz,Robert D., El Toro 92630 (CA)
(74) Representative: Froud, Clive

(56) References cited:
- BE-A- 556 977
- FR-A- 2 225 171
- FEDERATION PROCEEDINGS, vol. 34, no. 6, May 1975, pages 1458-1460; W. MOK et al.: "Cross-linked hemoglobins as potential plasma protein extenders"
- THE JOURNAL OF IMMUNOLOGY, vol. 137, no. 7, 1st October 1986, pages 2069-2072, The American Association of Immunologists, US; P. PEREZ et al.: "Specific lysis of human tumor cells by T cells coated with anti-T3 cross-linked to anti-tumor antibody"

## Description

The present invention relates to a novel process for chemically modifying biologically active materials in a controlled, continuous, and reproducible manner. The chemical reaction occurs in a dynamic, liquid flow reactor having at least two port means. By controlling the introduction sites for modifying reagents and quenchers in conjunction with the rate of the flow, one can reproducibly alter the weight, size, or functionality of a biologically active material in accordance with molecular weight distribution ranges that would not have been possible with prior methods. The present process is especially suited for producing polymerized hemoglobin with oxygen transport capacity.

As will be more fully described below, the present invention provides a process for the production of active intra-and inter-molecularly crosslinked hemoglobin polymers.

In accordance with the present invention, active intramolecularly crosslinked hemoglobin may first be produced by a process which comprises:
(a) flowing a defined volume of a hemoglobin-containing liquid through a reactor having an inlet means, an outlet means, at least one reaction port means, at least one quenching port means and a reaction zone disposed between the two, the liquid flow being at a substantially uniform rate and under pH, temperature and pressure conditions such that the activity of the hemoglobin is maintained;
(b) introducing a predetermined amount of at least one intramolecular crosslinking agent into the liquid flow through the reaction port means, thereby turbulently mixing the intramolecular crosslinking agent and the hemoglobin so as to form an intramolecularly crosslinked hemoglobin; and
(c) introducing a substantial excess of a quenching material into the liquid flow through the quenching port means, the quenching port means being disposed in relation to the reaction port means and the reaction zone such that the formation of the intramolecularly crosslinked hemoglobin is quenched in a substantially simultaneous manner after a predetermined period.

Further in accordance with the present invention, such may then be intermolecularly crosslinked by a process which comprises:
(a') flowing a defined volume of the liquid containing the intramolecularly crosslinked hemoglobin through a second reactor, having a second inlet means, a second outlet means, at least one second reaction port means, at least one second quenching port means, and a second reaction zone disposed between the two, the liquid flow being at a substantially uniform rate and under pH, temperature and pressure conditions such that the activity of the intramolecularly crosslinked hemoglobin is maintained;
(b') introducing a predetermined amount of at least one intermolecular crosslinking agent into the liquid flow through the second reaction port means, thereby turbulently mixing the intramolecularly crosslinked hemoglobin and the intermolecular crosslinking agent so as to form an intramolecularly and intermolecularly crosslinked hemoglobin; and
(c') introducing a substantial excess of a second quenching material into the liquid flow through the second quenching port means, the second quenching port means being disposed in relation to the second reaction port means and the second reaction zone such that the formation of the intramolecularly and intermolecularly crosslinked hemoglobin is quenched in a substantially simultaneous manner after a predetermined period.

The first stage of the present process provides a stable, active hemoglobin characterised in that it comprises a globular conformation of intramolecularly crosslinked, tetrameric hemoglobin subunits with greater than 90% of the molecules of the hemoglobin having a molecular weight of about 64,000 daltons.

The further stage of the present process provides a polyhemoglobin suitable for human use characterised in that it comprises a globular conformation of intermolecularly and intramolecularly crosslinked, tetrameric hemoglobin subunits, wherein the molecular weight of the polyhemoglobin is distributed between 130,000 and 780,000 daltons, with greater than 90% of the molecules of the polyhemoglobin having a molecular weight of about 390,000 daltons.

In the two stages of the present process, it is preferred that the predetermined amount of the intramolecular crosslinking agent or the intermolecular crosslinking agent is in a kinetic excess with respect to the hemoglobin or the intramolecularly crosslinked hemoglobin. Preferably, the quenching material, such as a lysine solution, changes the intramolecular crosslinking agent or the intermolecular crosslinking agent into a substantially inert substance. For the present purposes, the hemoglobin is generally from 2 to 8% of the reaction mixture. In the first stage of the present process, the hemoglobin may be conformationally stabilized with a stabilizing agent, preferably cyanide ion or carbon monoxide, in a reversible manner before being exposed to the intramolecular crosslinking agent, the stabilizing agent being removed after the reaction is quenched. Generally, the intramolecular crosslinking agent has a carbon chain length of between 6 and 12, such as a diimidate ester, or the intermolecular crosslinking agent has a carbon chain length of between 1 and 6, such as dialdehyde, preferably the intermolecular crosslinking agent having a carbon chain length less than that of the intramolecular crosslinking agent.

Having indicated some preferred features of the two aspects of the present invention, it will now be described more generally.

The need to modify biologically active materials into polymers has been especially felt in the field of hemoglobin blood substitutes. During the past twenty years, many different methods have been disclosed which require that a hemoglobin tetramer be chemically modified either through intermolecular or intramolecular crosslinking in order to be useful as a blood substitute.

In U.S. 3,925,344, Mazur discloses a process to polymerize hemoglobin intermolecularly by using dialkyl imidate esters as crosslinkers. Unfortunately, the average molecular weight was distributed from 65,000 to 600,000 daltons. In other words, the polyhemoglobin comprised a number of crosslinked polymers which did not have any specific configuration or size. (For the purposes of this description, "polyhemoglobin" refers to a molecule comprising more than one hemoglobin tetramer subunit.)

The uncontrolled nature of prior biopolymerization reactions can probably be traced to the batch methods which have been used. Temperature, pH, and most importantly, modifying reagant concentration and reaction time have been regulated only on a crude, macro level. The result has been polymerized products with extremely wide molecular weight distribution ranges.

The three fundamental steps of the process are interrrelated by the process reactor. By controlling the introduction sites and amounts of modifying reagants and quenchers in conjunction with the rate of the flow, one can reproducibly alter the weight, size, or functionality of the biologically active material to attain a narrow molecular weight distribution not previously achieveable. Following the flow through the reactor, a continuous stream of a liquid containing a biologically active material is flowed through a reactor, having at least a reaction port means, a quenching port means, and a reaction zone having a defined volume disposed between the two; said liquid flow being at a substantially uniform rate under pH, temperature, and pressure conditions that are compatible with maintaining the activity of the biologically active material. Then, at a predetermined rate, a defined amount of at least one modifying reagent and possibly a second biologically active material is introduced into the liquid flow through the reaction port means, thereby turbulently mixing the modifying reagent and the biologically active material so as to form a modified, biologically active material. Finally, a substantial excess of a quenching material is introduced into the liquid flow downstream of the reaction zone through the quenching port means, thereby quenching the formation of the modified, biologically active material in a controlled and substantially simultaneous manner.

Typically, the modifying reagent is kinetically optimized to produce a rapid reaction. Obviously, the reaction should reach the desired degree of modification before the flow exits the reaction zone. Likewise, kinetics plays an important role in the selection of a quenching material for the particular modifying reagent used. Preferably, one selects a material that can rapidly stop the reaction either by consuming the modifying reagent or changing either it or the biologically active material into an non-reactive form.

In practice, the present process produces polyhemoglobin in a continuous, large scale manner that is suitable for the production of a blood substitute on a commercial basis. The polyhemoglobin is uniform in that it reproducibly has a discrete conformation and molecular weight. Moreover, the present process does not result in any significant degradation of the hemoglobin during the transformation. Thus, more usable product with a higher oxygen transport capacity is obtained from the same amount of starting hemoglobin. For the first time the reaction conditions can be controlled so as to prevent unwanted modifying reactions such as intramolecular crosslinking during a intermolecular crosslinking reaction or excessive crosslinking during either procedure.

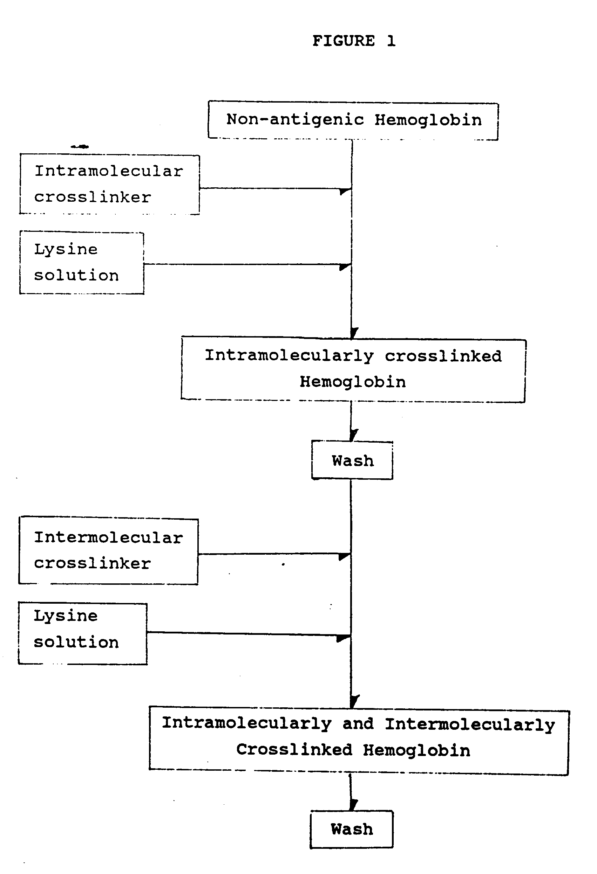

FIGURE 1 is a diagram of a reactor suitable for use in the present process.

FIGURE 2 is a block diagram of the production of a polyhemoglobin by the present process.

In the prior art processes, the presently-obtainable narrow molecular weight distribution range would have been impossible, typically ranging in excess of 300,000 daltons. However, the unique nature of the present continuous process permits a controlled polymerization of the biologically active molecules, i.e., polymers made from hemoglobin.

The present process reactor design enables an extremely controlled reaction between the biologically active material and the modifying reagants. Temperature, time, pH, pressure, and modifying reagent concentrations are controlled and reproducible. Thus, the reaction kinetics and the resultant products of the biopolymerization reaction can be contained within narrow, defined boundaries.

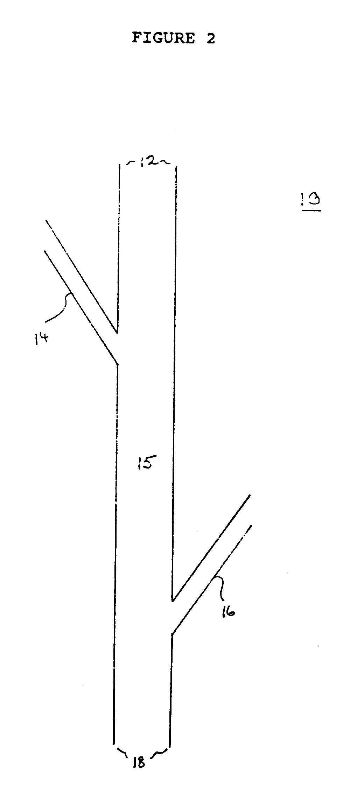

The key parameter of the reactor is the dimensions of the reaction zone with respect to those of all of the other reactor elements. As shown in Figure 2, the present reactor 10 comprises a sequence of an inlet means 12, a reaction port means 14, a quenching port means 16 and an outlet means 18. Between the reaction port means and the quenching port means is a reaction zone 15 having a predetermined volume and length. First, one must dimension the reaction zone such that at the designed flow throughput, the combined volumes of the mixed liquid flow from both the inlet means and the reaction port means will have time to react in accordance with the desired kinetics of the reaction before reaching the quenching port means. Second, one must dimension the quenching port and the outlet means such that any unreacted amounts of modifying reagents from the reaction zone flow can be mixed with an amount of quenching materials which is in an excess concentration sufficient to quench the reaction in a substantially simultaneous manner. Thus, a process reactor will differ in accordance with the particular kinetics of the desired reaction.

In the particular case of producing a polyhemoglobin, the reactor can be fashioned from stainless steel tubing having a diameter of about 0.4 to 0.8 cm. An orifice at the entry to the reaction zone restricts the amount of hemoglobin that is available for reaction in the reaction zone, in this case below 10¹⁶ molecules. The port means communicate with the flow path, meeting at an acute angle with respect thereto, preferably between about 30 and 60°. Metering through the ports may be either through the use of conventional pumps or through the use of the Venturi effect from the pumped hemoglobin flow. In the case of the Venturi effect, the ports should be of a smaller diameter than that of the reaction zone, preferably not exceeding about 70% thereof. In the case of heat-sensitive reactions or materials, heat exchangers may be disposed about the inlet means, the reaction zone, and the outlet means.

The hemoglobin to be used in making a blood substitute should be non-antigenic. The disclosure of how to obtain such a material is disclosed in a related patent application. However, for the purposes of illustration, conventional stroma-free hemoglobin can be used. Preferably, a stroma-free, bovine hemoglobin solution of about 7% is obtained which is buffered at about pH 7.6 to 8.0. Substantially all (e.g., 90%) of the hemoglobin molecules should be in a 64,000 dalton, tetrameric state with a stabilizing ligand attached.

Prior to reaction, an affinity reagent is added to the hemoglobin solution to further stabilize the hemoglobin. (For the purposes of this embodiment, an "affinity reagent" is a molecule which changes the ability of hemoglobin to load or off-load oxygen.) A suitable compound known to the art is pyridoxyl-5-phosphate (P5P), which is described in U.S. 4,529,719, to Tye. By reducing the oxygen affinity of the hemoglobin tetramer, the affinity molecule renders the hemoglobin more capable of supplying oxygen to human tissues.

A preferred form of hemoglobin for use in blood substitutes is intermolecularly and intramolecularly crosslinked. The present process enables one to separate these reactions, controlling each one so as to prevent the other. Thus, one can intramolecularly crosslink hemoglobin to the exclusion of intermolecular crosslinking. Suitable intramolecular crosslinking agents include homobifunctional or heterobifunctional compounds having two reactive sites that can bond between amino acid residues of the globin chains of hemoglobin, for example, diimidate esters and dialdehydes. (See U. S. 4,473,496 to Scannon, U. S. 4,061,736 to Morris et al., or U. S. 3,925,344 to Mazur.)

While many compounds are suitable for crosslinking, the carbon chain length between the two functional groups (i.e., reactive sites) of the crosslinking agent dictates the suitability for intramolecular versus intermolecular use. Preferably, the crosslinking agents are selected to bind to any of 46 lysine residues in hemoglobin. However, with the present process, one can limit the binding to less than 16 of those residues, preferably only 8. In doing so, one maintains the quaternary structure of hemoglobin which is responsible for oxygen transport characteristics. As is known in the art, in general, the purpose of crosslinking hemoglobin is to prevent disassociation into alpha-beta globin pairs. But, with the uncontrolled number of Intramolecular crosslinks found in the prior art, the natural conformational changes in the quaternary structure of hemoglobin needed for oxygen transport become hindered.

Intramolecular crosslinking is optimally achieved under the following conditions. First, a non-antigenic hemoglobin solution (about 1 to 5%) is pumped through the inlet means of a first reactor 10, as described above, and into the reaction zone 15. At the same time, a crosslinking agent having a carbon chain length of Cₓ, (where x represents the number of carbon atoms between the reactive ends of the molecule, and x is between 6 and 12), preferably a C₈ diimidate ester, is pumped into the reaction zone from the reaction port means 14. The intramolecular crosslinking agent is in a kinetic excess with respect to the hemoglobin. The reaction port means creates a Venturi effect which thoroughly mixes the two.

Almost simultaneously, a quenching solution is introduced into the reactor through the quenching port means 16, (preferably a 5% lysine hydrochloride solution or some other solution having a lysine molar concentration greater than that of the hemoglobin). The excess of lysine in the quenching solution drives the kinetics of the reaction, preferentially toward the lysine/crosslinking agent reaction, preventing further reaction of the crosslinking agent with the hemoglobin.

A high temperature heat exchanger of conventional design is disposed about the reactor so as to maintain the reaction zone at a temperature of between 35 and 50°C, but at least in excess of 30°C. The rate of flow of the hemoglobin solution through the reaction zone is controlled by a pump that maintains the hemoglobin under a pressure in excess of one atmosphere, preferably under five atmospheres. The controlled rate of flow through the reactor limits the reaction volume to between about 0.1 to 2.0 cm³ per second.

The hydrostatic pressure of the flow in the reaction zone should be optimized to maintain a Reynolds number which is greater than 3000 so as to maintain turbulence within the reaction zone. The result is that the crosslinking occurs substantially instantaneously, preferably occurring within about 0.03 to 0.60 seconds. Thus, the present process has a reproducible and consistent resident volume per time paramenter. The result is that 99% of all hemoglobin tetramers are intramolecularly crosslinked with less than 1% intermolecular crosslinking.

As the hemoglobin exits the reactor through the outlet means 18, a low temperature heat exchanger disposed thereabout lowers the temperature of the hemoglobin solution to about 4°C. The intramolecularly crosslinked hemoglobin solution is flowed into a first washing means where excess lysine and polylysine is removed by conventional methods, for example, ultrafiltration. The intramolecularly crosslinked hemoglobin solution is concentrated by the ultrafiltration to a concentration of about 6 to 12% before being pumped into the inlet means of a second reactor where it can now be intermolecularly crosslinked as well.

Unlike the first reactor, the second one includes a plurality of reaction zones, with respective inlet means, reaction port means, quenching port means, and outlet means. Again, the reactor is made of stainless steel tubing of about 0.125 mm in diameter with a plurality of reaction zones. By having a number of reaction zones the rate of flow of hemoglobin solution through the second reactor is equal to the output from the first reactor while reducing the effective amount of hemoglobin solution exposed to an intermolecular crosslinking agent in the reaction zone.

The intermolecular crosslinking agent having a carbon chain length of C_{y}, (where y represents the number of carbon atoms between the reactive ends of the molecule and y is less than 6), preferably glutaraldehyde, and reacts with about only 4 to 8 lysine residues. Almost simultaneously, it is pumped into the reaction zones from the reaction port means. The intermolecular crosslinking agent is in kinetic excess with respect to the hemoglobin. The plurality of reaction port means create a Venturi effect in the reaction zones which thoroughly mixes the two materials therein.

Almost simultaneously, a quenching solution is introduced into the reactor through the respective quenching port means 16, (preferably a 5% lysine hydrochloride solution or some other solution having a lysine molar concentration greater than that of the hemoglobin). The excess of lysine in the quenching solution drives the kinetics of the reaction preferentially toward the lysine/crosslinking agent reaction, preventing further reaction of the crosslinking agent with the hemoglobin.

A high temperature heat exchanger of conventional design is disposed about the reactor so as to maintain the reaction zone at a temperature of between 35 and 50°C, but at least in excess of 30°C. The rate of flow of the hemoglobin solution through the reaction zone is controlled by a pump that maintains the hemoglobin under a hydrostatic pressure in excess of one atmosphere, preferably under five atmospheres. The controlled rate of flow through the reactor limits the reaction volume to between about 0.004 to 0.100 cm³ per second.

The hydrostatic pressure of the flow in the reaction zones should be optimized as in the first reactor to maintain turbulence therein. Again, crosslinking occurs substantially instantaneously, preferably occurring within about 0.03 to 0.60 seconds. Thus, the present process has a reproducible and consistent resident volume per time parameter. The result is that 99% of all hemoglobin tetramers are intermolecularly crosslinked with less than 1% being further intramolecularly crosslinked.

As the hemoglobin exits the reactor through the outlet means 18, a low temperature heat exchanger disposed thereabout, lowers the temperature of the hemoglobin solution to about 4°C. The intramolecularly and intermolecularly crosslinked hemoglobin solution is flowed into a second washing means where excess lysine from the quenching solution is removed by conventional methods, for example, ultrafiltration, where again it can be concentrated to an about 10% polyhemoglobin solution.

As can be appreciated by those skilled in the art, the number of lysine residues that are bonded can be altered by appropriately changing the reaction time and relative concentrations of crosslinking agents to hemoglobin. By either decreasing or increasing either of these parameters, the extent of crosslinking is either decreased or increased, respectively. The resulting polyhemoglobin has a molecular weight distribution between 130.000 and 780,000 daltons, with greater that 90% of the molecules in the 390,000 dalton form of a globular, hexameric struture, at a a concentration of about 5 to 15 grams per 100 milliliters. Prior art processes could not even achieve 50% of the yield in this particular form.

Final treatments of the solution include known methods of filtering to remove unwanted molecular weight fractions and equilibration with a physiologically acceptable fluid such as a renal dialysis fluid. In addition if the hemoglobin has been stabilized or modified by ligands into a non-oxyhemoglobin form, the hemes of the polyhemoglobin are returned to oxygenated forms by conventional means. In this form the polyhemglobin can be used either in vivo as an oxygen transport fluid or a plasma expander, or it can be used in vitro as an organ perfusion fluid. Furthermore, the polyhemoglobin is characterized by a P₅₀ of about 22 to 30 mm Hg, an oncotic pressure of about 25 mm Hg, a pH of about 7.4, a sodium concentration of about 140 to 150 mEq/L, a potassium concentration of about 3.0 to 4.5 mEq/L, an oxygen content of greater than 90% saturation, and a shelf life of greater than one year when stored at 2 to 8°C. Such a polyhemoglobin is the first to combine the properties of an oxygen transport capacity similar to erythrocytes, ans oncotic strength sufficient to maintain plasma volume, non-antigenicity, and a suitably long shelf life.

## Claims

1. A process for the production of active intra-molecularly crosslinked hemoglobin characterised in that it comprises:
(a) flowing a defined volume of a hemoglobin-containing liquid through a reactor having an inlet means, an outlet means, at least one reaction port means, at least one quenching port means and a reaction zone disposed between the two, the liquid flow being at a substantially uniform rate and under pH, temperature and pressure conditions such that the activity of the hemoglobin is maintained;
(b) introducing a predetermined amount of at least one intramolecular crosslinking agent into the liquid flow through the reaction port means, thereby turbulently mixing the intramolecular crosslinking agent and the hemoglobin so as to form an intramolecularly crosslinked hemoglobin; and
(c) introducing a substantial excess of a quenching material into the liquid flow through the quenching port means, the quenching port means being disposed in relation to the reaction port means and the reaction zone such that the formation of the intramolecularly crosslinked hemoglobin is quenched in a substantially simultaneous manner after a predetermined period.

2. A process for the intermolecular crosslinking of active intramolecularly crosslinked hemoglobin obtainable by a process as claimed in claim 1 characterised in that it further comprises:
(a') flowing a defined volume of the liquid containing the intramolecularly crosslinked hemoglobin through a second reactor, having a second inlet means, a second outlet means, at least one second reaction port means, at least one second quenching port means, and a second reaction zone disposed between the two, the liquid flow being at a substantially uniform rate and under pH, temperature and pressure conditions such that the activity of the intramolecularly crosslinked hemoglobin is maintained;
(b') introducing a predetermined amount of at least one intermolecular crosslinking agent into the liquid flow through the second reaction port means, thereby turbulently mixing the intramolecularly crosslinked hemoglobin and the intermolecular crosslinking agent so as to form an intramolecularly and intermolecularly crosslinked hemoglobin; and
(c') introducing a substantial excess of a second quenching material into the liquid flow through the second quenching port means, the second quenching port means being disposed in relation to the second reaction port means and the second reaction zone such that the formation of the intramolecularly and intermolecularly crosslinked hemoglobin is quenched in a substantially simultaneous manner after a predetermined period.

3. A process as claimed in claim 1 or claim 2 wherein the predetermined amount of the intramolecular crosslinking agent or the intermolecular crosslinking agent is in a kinetic excess with respect to the hemoglobin or the intramolecularly crosslinked hemoglobin.

4. A process as claimed in any of claims 1 to 3 wherein the quenching material, such as a lysine solution, changes the intramolecular crosslinking agent or the intermolecular crosslinking agent into a substantially inert substance.

5. A process as claimed in any of claims 1 to 4 wherein the hemoglobin is from 2 to 8% of the reaction mixture.

6. A process as claimed in any of claims 1 or 3 to 5 wherein the hemoglobin is conformationally stabilized with a stabilizing agent, preferably cyanide ion or carbon monoxide, in a reversible manner before being exposed to the intramolecular crosslinking agent, the stabilizing agent being removed after the reaction is quenched.

7. A process as claimed in any of claims 1 to 6 wherein the rate and volume of the flow of the liquid through the reactor is from 0.1 to 2.0 cm per second.

8. A process as claimed in any of claims 1 to 7 wherein the intramolecular crosslinking agent has a carbon chain length of between 6 and 12, such as a diimidate ester, or the intermolecular crosslinking agent has a carbon chain length of between 1 and 6, such as a dialdehyde, preferably the intermolecular crosslinking agent having a carbon chain length less than that of the intramolecular crosslinking agent.

9. A stable, active intramolecularly crosslinked hemoglobin characterised in that it comprises a globular conformation of intramolecularly crosslinked, tetrameric hemoglobin subunits with greater than 90% of the molecules having a molecular weight of about 64,000 daltons, which intramolecularly crosslinked hemoglobin is obtainable by a process as claimed in any of claims 1 or 3 to 7.

10. A polyhemoglobin suitable for human use characterised in that it comprises a globular conformation of intermolecularly and intramolecularly crosslinked, tetrameric hemoglobin subunits, wherein the molecular weight of the polyhemoglobin is distributed between 130,000 and 780,000 daltons, with greater than 90% of the molecules of the polyhemoglobin having a molecular weight of about 390,000 daltons, which polyhemoglobin is obtainable by a process as claimed in any of claims 1 to 8.

## Patentansprüche

1. Verfahren zur Herstellung eines aktiven, intramolekular vernetzten Hämoglobins,
**dadurch gekennzeichnet,**
daß es die nachfolgenden Schritte umfaßt:
(a) Fließen eines definierten Volumens einer Hämoglobinenthaltenden Flüssigkeit durch einen Reaktor mit einem Einlaßmittel, einem Auslaßmittel, wenigstens einem Reaktionsöffnungsmittel, wenigstens einem Quenchingöffnungsmittel und einer Reaktionszone, die zwischen den beiden angeordnet ist, wobei der Flüssigkeitsstrom mit einer im wesentlichen gleichbleibenden Geschwindigkeit und unter pH-, Temperatur- und Druckbedingungen erfolgt, so daß die Aktivität des Hämoglobins erhalten bleibt;
(b) Einführen einer vorherbestimmten Menge wenigstens eines Mittels zur intramolekularen Vernetzung in den Flüssigkeitsstrom durch das Reaktionsöffnungsmittel, wodurch das Mittel zur intramolekularen Vernetzung und das Hämoglobin heftig vermischt werden, um ein intramolekular vernetztes Hämoglobin zu bilden; und
(c) Einführen eines wesentlichen Überschusses eines Quenchingmaterials in den Flüssigkeitsstrom durch das Quenchingöffnungsmittel, wobei das Quenchingöffnungsmittel in Bezug auf das Reaktionsöffnungsmittel und die Reaktionszone so angeordnet ist, daß die Bildung des intramolekular vernetzten Hämoglobins in einer im wesentlichen gleichzeitigen Weise nach einer vorherbestimmten Zeitdauer gequencht wird.

2. Verfahren zur intermolekularen Vernetzung von aktivem intramolekular vernetztem Hämoglobin, welches durch ein Verfahren nach Anspruch 1 erhältlich ist,
dadurch gekennzeichnet,
daß es weiterhin die nachfolgenden Schritte umfaßt:
(a') Fließen eines definierten Volumens der das intramolekular vernetzte Hämoglobin enthaltenden Flüssigkeit durch einen zweiten Reaktor, welcher ein zweites Einlaßmittel, ein zweites Auslaßmittel, wenigstens ein zweites Reaktionsöffnungsmittel, wenigstens ein zweites Quenchingöffnungsmittel und eine zweite Reaktionszone enthält, die zwischen den beiden angeordnet ist, wobei der Flüssigkeitsstrom mit einer im wesentlichen gleichbleibenden Geschwindigkeit und unter pH-, Temperatur- und Druckbedingungen erfolgt, so daß die Aktivität des intramolekular vernetzten Hämoglobins erhalten bleibt;
(b') Einführen einer vorherbestimmten Menge wenigstens eines intermolekular vernetzten Mittels in den Flüssigkeitsstrom durch das zweite Reaktionsöffnungsmittel, wodurch das intramolekular vernetzte Hämoglobin und das Mittel zur intermolekularen Vernetzung heftig vermischt werden, so daß ein intramolekular und intermolekular vernetztes Hämoglobin gebildet wird; und
(c) Einführen eines wesentlichen Überschusses eines zweiten Quenchingmaterials in den Flüssigkeitsstrom durch das zweite Quenchingöffnungsmittel, wobei das zweite Quenchingöffnungsmittel im Verhältnis zum zweiten Reaktionsöffnungsmittel und zur zweiten Reaktionszone so angeordnet ist, daß die Bildung des intramolekular und intermolekular vernetzten Hämoglobins in einer im wesentlichen gleichzeitigen Weise nach einer vorherbestimmten Zeitdauer gequencht wird.

3. Verfahren nach Anspruch 1 oder 2, wobei die vorherbestimmte Menge des Mittels zur intramolekularen Vernetzung oder des Mittels zur intermolekularen Vernetzung in einem kinetischen Überschuß in Bezug auf das Hämoglobin oder das intramolekular vernetzte Hämoglobin vorliegt.

4. Verfahren nach einem der Ansprüche 1 bis 3, wobei das Quenchingmaterial, beispielsweise eine Lysinlösung, das Mittel zur intramolekularen Vernetzung oder das Mittel zur intermolekularen Vernetzung in einen im wesentlichen inerten Stoff umwandelt.

5. Verfahren nach einem der Ansprüche 1 bis 4, wobei das Hämoglobin 2 bis 8 % der Reaktionsmischung ausmacht.

6. Verfahren nach einem der Ansprüche 1 oder 3 bis 5, wobei das Hämoglobin mit einem Stabilisierungsmittel, bevorzugt einem Cyanidion oder Kohlenmonoxid, in einer reversiblen Weise in seiner Konformation stabilisiert wird, bevor es dem Mittel zur intramolekularen Vernetzung ausgesetzt wird, wobei das Stabilisierungsmittel entfernt wird, nachdem die Reaktion gequencht wurde.

7. Verfahren nach einem der Ansprüche 1 bis 6, wobei die Geschwindigkeit und das Volumen des Flüssigkeitsstroms durch den Reaktor von 0,1 bis 2,0 cm/sek beträgt.

8. Verfahren nach einem der Ansprüche 1 bis 7, wobei das Mittel zur intramolekularen Vernetzung eine Kohlenstoffkettenlänge zwischen 6 und 12, beispielsweise ein Diimidatester, aufweist, oder das Mittel zur intermolekularen Vernetzung eine Kohlenstoffkettenlänge zwischen 1 und 6, beispielsweise ein Dialdehyd, aufweist, wobei das Mittel zur intermolekularen Vernetzung bevorzugt eine Kohlenstoffkettenlänge aufweist, die geringer ist als die des Mittels zur intramolekularen Vernetzung.

9. Stabiles, aktives intramolekular vernetztes Hämoglobin,
**dadurch gekennzeichnet,**
daß es eine globulare Konformation der intramolekular vernetzten, tetrameren Hämoglobinuntereinheiten aufweist, wobei mehr als 90 % der Moleküle ein Molekulargewicht von etwa 64.000 Dalton aufweist, und das intramolekular vernetzte Hämoglobin durch ein Verfahren nach einem der Ansprüche 1 oder 3 bis 7 erhältlich ist.

10. Zur Verwendung beim Menschen geeignetes Polyhämoglobin,
dadurch gekennzeichnet,
daß es eine globulare Konformation der intermolekular und intramolekular vernetzten tetrameren Hämoglobinuntereinheiten aufweist, wobei das Molekulargewicht des Polyhämoglobins zwischen 130.000 und 780.000 Dalton verteilt ist, wobei mehr als 90 % der Polyhämoglobinmoleküle ein Molekulargewicht von etwa 390.000 Dalton aufweisen, und wobei das Polyhämoglobin durch ein Verfahren nach einem der Ansprüche 1 bis 8 erhältlich ist.

## Revendications

1. Procédé pour la production d'hémoglobine active réticulée intramoléculairement, caractérisé en ce qu'il comprend:
(a) l'écoulement d'un volume défini d'un liquide contenant de l'hémoglobine à travers un réacteur ayant un moyen d'alimentation, un moyen de soutirage, au moins un orifice de réaction, au moins un orifice de trempe et une zone de réaction disposée entre les deux, l'écoulement du liquide étant effectué à une vitesse sensiblement uniforme et dans des conditions de pH, de température et de pression telles que l'activité de l'hémoglobine soit maintenue;
(b) l'introduction d'une quantité prédéterminée d'au moins un agent de réticulation intramoléculaire dans l'écoulement du liquide à travers l'orifice de réaction, mélangeant ainsi de façon turbulente l'agent de réticulation intramoléculaire et l'hémoglobine afin de former une hémoglobine réticulée intramoléculairement; et
(c) l'introduction d'un excès important d'un agent de trempe dans l' écoulement du liquide à travers l'orifice de trempe, l'orifice de trempe étant disposé par rapport à l'orifice de réaction et à la zone de réaction de sorte que la formation de l' hémoglobine réticulée intramoléculairement soit trempée d'une manière quasi-simultanée après une durée prédéterminée.

2. Procédé de réticulation intermoléculaire d'hémoglobine active réticulée intramoléculairement que l'on peut obtenir par un procédé selon la revendication 1, caractérisé en ce qu'il comprend de plus:
(a') l'écoulement d'un volume défini du liquide contenant l'hémoglobine réticulée intramoléculairement à travers un second réacteur, ayant un second moyen d'alimentation, un second moyen de soutirage, au moins un deuxième orifice de réaction, au moins un deuxième orifice de trempe, et une zone de réaction disposée entre les deux, l'écoulement du liquide étant à un débit sensiblement uniforme et dans des conditions de pression, de température et de pH telles que l'activité de l'hémoglobine réticulée intramoléculairement soit maintenue;
(b') l'introduction d'une quantité prédéterminée d'au moins un agent de réticulation intermoléculaire dans l'écoulement du liquide à travers le deuxième orifice de réaction, mélangeant ainsi de façon turbulente l'hémoglobine réticulée intramoléculairement et l'agent de réticulation intermoléculaire de façon à former une hémoglobine réticulée intramoléculairement et intermoléculairement; et
(c') l'introduction d'un excès important d'un deuxième agent de trempe dans l'écoulement liquide à travers le deuxième orifice de trempe, le deuxième orifice de trempe étant disposé par rapport au deuxième orifice de réaction et la seconde zone de réaction de sorte que la formation de l'hémoglobine réticulée intramoléculairement et intermoléculairement soit trempée de façon quasi-simultanée après une durée prédéterminée.

3. Procédé selon la revendication 1 ou 2, dans lequel la quantité prédéterminée de l'agent de réticulation intramoléculaire ou de l'agent de réticulation intermoléculaire est en excès cinétique par rapport à l'hémoglobine ou à l'hémoglobine réticulée intramoléculairement.

4. Procédé selon l'une quelconque des revendications 1 à 3, dans lequel l'agent de trempe, telle qu'une solution de lysine, modifie l'agent de réticulation intramoléculaire ou l'agent de réticulation intermoléculaire en une substance sensiblement inerte.

5. Procédé selon l'une quelconque des revendications 1 à 4, dans lequel l'hémoglobine représente 2 à 8% du mélange réactionnel.

6. Procédé selon l'une quelconque des revendications 1 ou 3 à 5, dans lequel l'hémoglobine est stabilisée en conformation avec un agent de stabilisation, de préférence un ion cyanure ou du monoxyde de carbone, d'une manière réversible avant d'être exposée à l'agent de réticulation intramoléculaire, l'agent de stabilisation étant éliminé après avoir trempé la réaction.

7. Procédé selon l'une quelconque des revendications 1 à 6, dans lequel le débit et le volume de l'écoulement du liquide à travers le réacteur est de 0,1 à 2,0 cm par seconde.

8. Procédé selon l'une quelconque des revendications 1 à 7, dans lequel l'agent de réticulation intramoléculaire possède une longueur de chaîne d'atomes de carbone comprise entre 6 et 12, tel qu'un ester diimidate, ou bien l'agent de réticulation intermoléculaire possède une longueur de chaîne d'atomes de carbone comprise entre 1 et 6, tel qu'un dialdéhyde, l'agent de réticulation intermoléculaire ayant, de préférence, une longueur de chaîne d'atomes de carbone inférieure à celle de l'agent de réticulation intramoléculaire.

9. Hémoglobine réticulée intramoléculairement, active, stable, caractérisée en ce qu'elle comprend une conformation globulaire de sous-motifs d'hémoglobine tétramères réticulés intramoléculairement avec plus de 90% des molécules ayant une masse moléculaire d'environ 64 000 daltons, hémoglobine réticulée intramoléculairement que l'on peut obtenir par un procédé selon l'une quelconque des revendications 1 ou 3 à 7.

10. Polyhémoglobine appropriée pour une utilisation humaine, caractérisée en ce qu'elle comprend une conformation globulaire de sous-motifs d'hémoglobine tétramères, réticulés intermoléculairement et intramoléculairement, dans lesquels la masse moléculaire de la polyhémoglobine est distribuée entre 130 000 et 780 000 daltons, avec plus de 90% des molécules de la polyhémoglobine ayant une masse moléculaire d'environ 390 000 daltons, polyhémoglobine que l'on peut obtenir par un procédé selon l'une quelconque des revendications 1 à 8.
